# EUROPEAN PATENT APPLICATION

(11) **EP 4 438 595 A1**
(43) Date of publication of application: **02.10.2024**
(21) Application number: 22966354.7
(22) Date of filing: 09.12.2022
(51) Int. Cl.: C07D 213/75, C07D 513/04

(54) **EDOXABAN KEY INTERMEDIATE AND SYNTHESIS METHOD THEREFOR**

(30) Priority: 24.11.2022 CN 202211480026
(71) Applicant: Shanghai Bios Technology Co., Ltd., Shanghai 201499 (CN)
(72) Inventor: PENG, Qiujun, Shanghai 201499 (CN); YU, Wansheng, Shanghai 201499 (CN); RUAN, Libo, Shanghai 201499 (CN); ZENG, Yifei, Shanghai 201499 (CN); LI, Qinqin, Shanghai 201499 (CN)
(74) Representative: Bayramoglu et al.
(86) International application number: PCT/CN2022/137889
(87) International publication number: WO 2024/108670

(57) **Abstract**

The present invention discloses an edoxaban key intermediate and a synthesis method therefor. The edoxaban key intermediate is 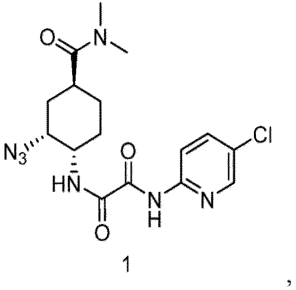 and is prepared by an ammonolysis reaction between raw materials. Edoxaban p-toluenesulfonate monohydrate can be prepared by using this intermediate. Edoxaban p-toluenesulfonate hydrate is prepared on an industrial scale by using a new method and a new route which are simple and efficient and can effectively reduce the cost.

## Description

### TECHNICAL FIELD

The present invention relates to an edoxaban key intermediate and a synthesis method therefor; and edoxaban p-toluenesulfonate monohydrate is further prepared from this key intermediate as a raw material.

### BACKGROUND

Edoxaban p-toluenesulfonate monohydrate is developed by Daiichi Sankyo Inc., is a small molecule drug, and is a coagulation factor Xa inhibitor. At present, the highest stage of research and development of this drug is approved for marketing for the treatment of pulmonary embolism, venous thromboembolism, stroke, venous thrombosis and embolism. On April 22, 2011, Edoxaban tosylate was approved by the Japan Pharmaceutical and Medical Device Agency (PMDA) and sold by Daiichi Sankyo Inc. On January 8, 2015, Edoxaban tosylate was approved by the U.S. Food and Drug Administration (FDA), and sold by Daiichi Sankyo Inc. under a trade name of Savaysa^{®}. (NDA206316) On June 19, 2015, Edoxaban tosylate was approved by the European Medicines Agency (EMA), and sold by Daiichi Sankyo Europe Gmbh under a trade name of Lixiana^{®}. (EMEA/H/C/002629) On December 25, 2018, Edoxaban tosylate was approved by the National Medical Products Administration (NMPA) of China, and sold by Daiichi Sankyo Europe Gmbh under a trade name of LIXIANA^{®}.

Edoxaban has three chiral centers and a total of 8 isomers, but only one configuration has good activity, and a structure of Edoxaban is as follows:

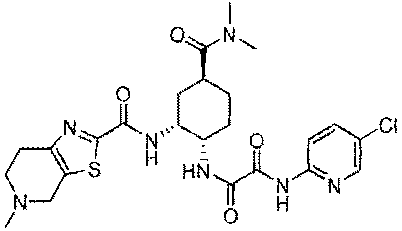

A synthetic route published in US2005119486A1 by JP Daiichi Sankyo Inc. is as follows:

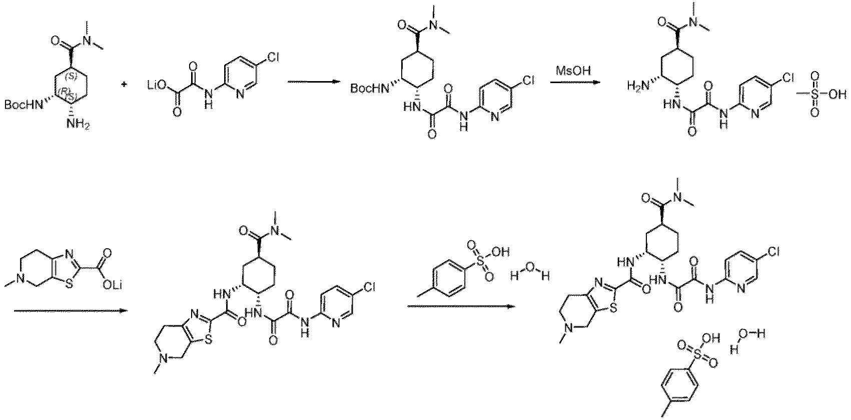

In this route, an amide is formed from a chiral amine compound and a lithium salt of an oxamide derivative under the action of a condensing agent; a Boc-protecting group is removed under acidic conditions; and an amide is then formed from the obtained product with the Boc-protecting group removed and a lithium salt of a 2-thiazolecarboxylic acid derivative in the presence of a condensing agent to prepare Edoxaban.

Another synthetic route is also published in US2005119486A1:

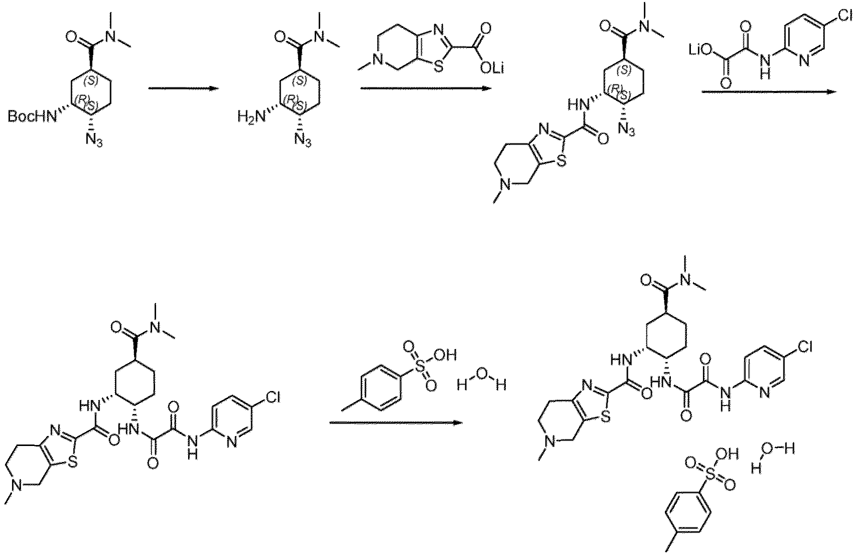

In this route, Boc protection is first removed from a chiral azide compound as a starting material, an amide is formed from the obtained product with Boc protection removed and a lithium salt of a 2-thiazolecarboxylic acid derivative in the presence of a condensing agent, azido is then reduced to amino, and an amide is formed from the obtained product and a lithium salt of an oxalamide derivative under the action of a condensing agent to prepare Edoxaban.

A synthetic route of Edoxaban published in WO2008156159A1 by JP Daiichi Sankyo Inc is shown below. Racemic S-1 is resolved in the presence of a chiral amine S-2, followed by intramolecular nucleophilic attack under the action of an electrophilic brominating reagent to obtain a bridged ring compound S-4. The S-4 is subjected to aminolysis, followed by ring opening under the action of NH₄OH through a propylene oxide intermediate S-6 to obtain an amino alcohol compound S-7. Then, amino in the S-7 is protected with Boc, alcoholic hydroxyl in the S-7 is protected with Ms, and NaN₃ attack is to introduce a N₃ group to obtain a compound S-10. Subsequently, reduction by hydrogenation is performed to obtain amino, and the obtained compound and S-13 are subjected to condensation to obtain a compound S-14. The Boc protecting group of the compound S-14 is removed under acidic conditions, and then the obtained compound and carboxylic acid S-16 are subjected to condensation to obtain a final compound S-17, which forms a TsOH salt to obtain edoxaban. A synthetic route of this process is as follows:

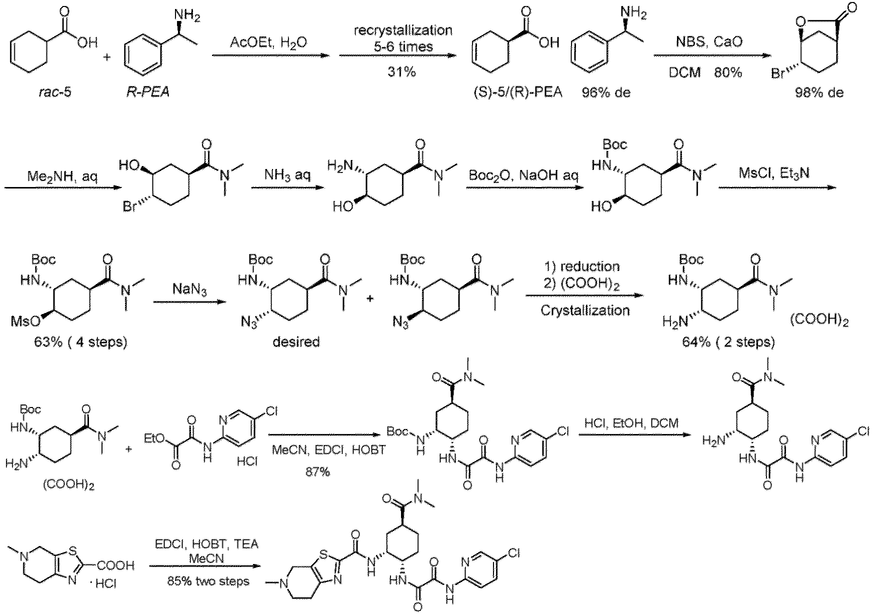

A difficulty in the synthesis of this compound lies in the preparation of chiral substrates such as the compound S-10.

### SUMMARY

The technical problem to be solved by the present invention is to provide a new key intermediate of Edoxaban and a preparation method therefor; and edoxaban p-toluenesulfonate monohydrate is further prepared from this key intermediate as a raw material.

In order to solve the above problem, the present invention adopts the following technical solutions:
an edoxaban key intermediate has a structural formula shown in a formula 1:

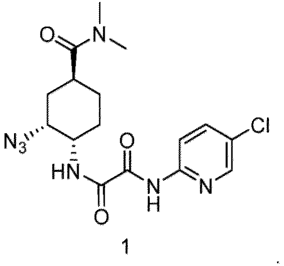

The present invention further provides a synthesis method for the edoxaban key intermediate, wherein the edoxaban key intermediate is obtained by an ammonolysis reaction between a compound of a formula 2 and a compound of a formula 3, and a synthetic route is as follows:

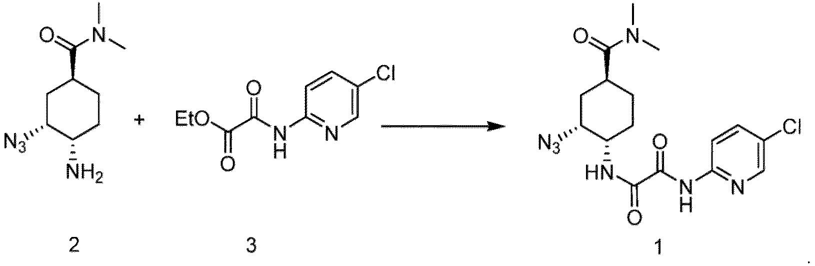

Preferably, a solvent used in the ammonolysis reaction is at least one of acetonitrile, tetrahydrofuran, ethanol, toluene, and butanone; and an alkali used in the ammonolysis reaction is at least one of triethylamine, diisopropylethylamine, pyridine, and DBU.

Preferably, the ammonolysis reaction is carried out at a temperature of 50-100°C.

Preferably, a molar ratio of the compound of the formula 2 to the compound of the formula 3 is 1:1.1-1.5.

The present invention further provides use of the edoxaban key intermediate, being used for the preparation of edoxaban p-toluenesulfonate monohydrate, specifically including the steps of:
Step 1): subjecting a compound of a formula 1 to a reduction reaction to obtain a compound of a formula 4, wherein a synthetic route is as follows: 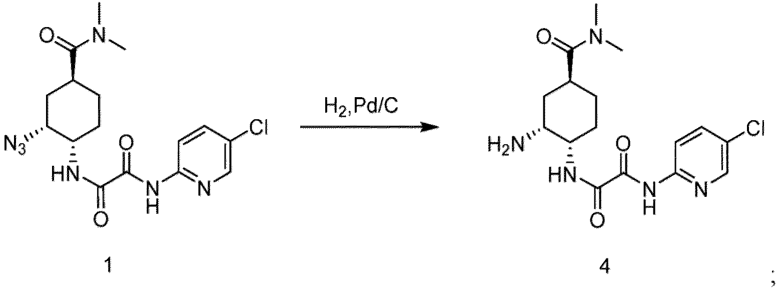
Step 2): subjecting the compound of the formula 4 to a condensation reaction to obtain a compound of a formula 6, wherein a synthetic route is as follows: 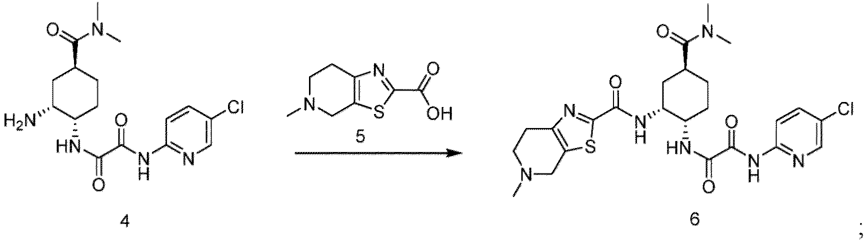 and
Step 3): performing salt formation by using the compound of the formula 6 and p-toluenesulfonic acid to ontain edoxaban p-toluenesulfonate monohydrate, i.e., a compound of a formula 7:

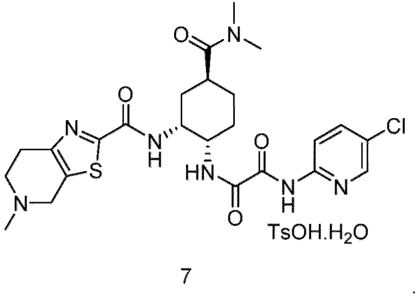

Preferably, a reducing agent used in the step 1) is at least one of Pd/C, Raney nickel, lithium aluminum hydride, sodium borohydride, and triphenylphosphine.

Preferably, a solvent used in the condensation reaction of the step 2) is at least one of acetonitrile, ethanol, tetrahydrofuran, and dichloromethane.

Preferably, in the step 2), a compound of a formula 5 is prepared into acyl chloride for the reaction or a condensation reagent is used for the reaction, the condensation reagent being at least one of CDI, DCC, and EDCI.

Preferably, the step 3) is carried out in a reaction solvent, and the reaction solvent is at least one of ethanol, acetonitrile, and acetone; the temperature is controlled to be 50-100°C in the step 3); and the temperature of the salt formation in the step 3) is -10°C to 30°C.

The present invention provides the new key intermediate of Edoxaban and the preparation method therefor; and edoxaban p-toluenesulfonate monohydrate is further prepared from from this key intermediate as a raw material, higher yield and chiral purity can be achieved, the reaction conditions are mild, the process operation is simple, and the method is suitable for industrial production.

### BRIEF DESCRIPTION OF THE DRAWINGS

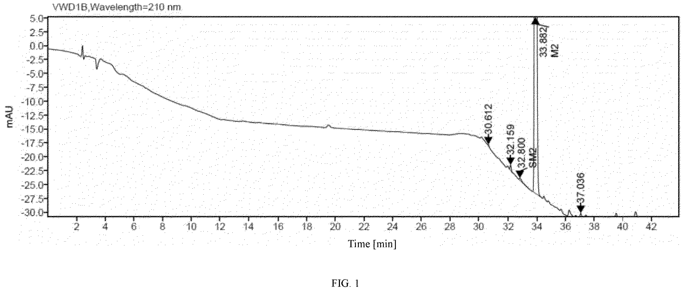

FIG. 1 is an HPLC spectrum of N1-((1S,2R,4S)-2-azido-4-(dimethylcarbamoyl)cyclohexyl)-N2-(5-chloropyridin-2-yl) oxalamide (a compound of a formula 1); and

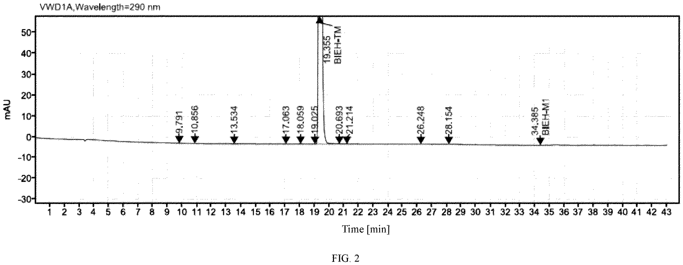

FIG. 2 is an HPLC spectrum of edoxaban p-toluenesulfonate monohydrate (a compound of a formula 7).

### DETAILED DESCRIPTION

In order to make the present invention more clearly understood, the present invention will be described in detail below by preferred examples with reference to the accompanying drawings.

### Example 1

### Preparation of N1-((1S,2R,4S)-2-azido-4-(dimethylcarbamoyl)cyclohexyl)-N2-(5-chloropyridin-2-yl) oxalamide (a compound of a formula 1)

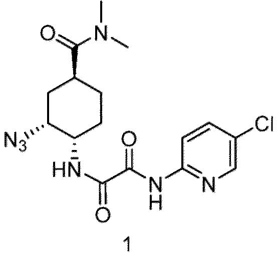

Ethyl 2-((5-chloropyridin-2-yl)amino)-2-oxoacetate (a compound of a formula 3) (150.7 g, 568.7 mmol), and 500 mL of ethanol were sequentially added into a reaction flask; and a reaction system was cooled to about 0-10°C, triethylamine (43.1 g, 426.7 mmol) was added slowly, (1S,3R,4S)-4-amino-3-azido-N,N-dimethylcyclohexane-1-carboxamide (a compound of a formula 2) (100.0 g, 473.9 mmol) was then added, after the addition was completed, the reaction system was heated to 70-75°C, when the system was heated to 55-60°C, triethylamine (14.4 g, 142.6 mmol) was added into the system, and stirring was then performed for 16 h while maintaining the temperature of the system at 70-75°C. After completion of the reaction by liquid phase monitoring, the system was cooled to 50-55°C, 900 mL of purified water was added dropwise, after the addition was completed, cooling was performed to 20-25°C, stirring was performed for 1 h, and suction filtration was performed to obtain 163.9 g of a compound (the compound of the formula 1), with a yield of 88.0%.

### H NMR data of the obtained product was as follows:

¹H NMR (400 MHz, CDCl₃) δ 9.79 (s, 1H), 8.32 (d, J = 2.4 Hz, 1H), 8.19 (d, J = 8.8 Hz, 1H), 7.72 (dd, J = 8.9, 2.4 Hz, 1H), 7.63 (d, J = 8.8 Hz, 1H), 4.13 (d, J = 3.4 Hz, 1H), 4.02 (td, J = 9.7, 8.9, 4.7 Hz, 1H), 3.09 (s, 3H), 2.95 (s, 3H), 2.93 - 2.84 (m, 1H), 2.04 (dtd, J = 23.4, 13.2, 11.4, 6.1 Hz, 2H), 1.83 (dt, J = 10.0, 5.9 Hz, 2H), 1.69 (d, J = 10.1 Hz, 2H).

¹³C NMR (101 MHz, CDCl₃) δ 173.92, 158.32, 157.58, 148.12, 147.17, 138.07, 128.02, 114.62, 60.42, 50.05, 37.17, 35.68, 33.47, 31.63, 27.27, 26.17.

LCMS: m/z=393.8 (M+H)⁺.

### Example 2

### Preparation of N1-((1S,2R,4S)-2-azido-4-(dimethylcarbamoyl)cyclohexyl)-N2-(5-chloropyridin-2-yl) oxalamide (a compound of a formula 1)

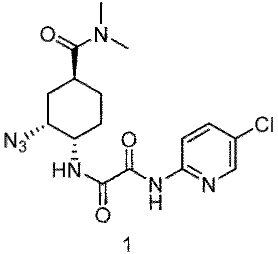

Ethyl 2-((5-chloropyridin-2-yl)amino)-2-oxoacetate (a compound of a formula 3) (150.7 g, 568.7 mmol), and 500 mL of tetrahydrofuran were sequentially added into a reaction flask; and a reaction system was cooled to about 0-10°C, triethylamine (43.1 g, 426.7 mmol) was added slowly, (1S,3R,4S)-4-amino-3-azido-N,N-dimethylcyclohexane-1-carboxamide (a compound of a formula 2) (100.0 g, 473.9 mmol) was then added, after the addition was completed, the reaction system was heated to 60-65°C, triethylamine (14.4 g, 142.6 mmol) was then added into the system, and stirring was then performed for 16 h while maintaining the temperature of the system at 60-65°C. After completion of the reaction by liquid phase monitoring, 900 mL of purified water was added dropwise to the system, after the addition was completed, cooling was performed to 20-25°C, stirring was performed for 1 h, and suction filtration was performed to obtain 167.6 g of a compound (the compound of the formula 1), with a yield of 90.0%.

H NMR data of the obtained product was as follows:
¹H NMR (400 MHz, CDCl₃) δ 9.79 (s, 1H), 8.32 (d, J = 2.4 Hz, 1H), 8.19 (d, J = 8.8 Hz, 1H), 7.72 (dd, J = 8.9, 2.4 Hz, 1H), 7.63 (d, J = 8.8 Hz, 1H), 4.13 (d, J = 3.4 Hz, 1H), 4.02 (td, J = 9.7, 8.9, 4.7 Hz, 1H), 3.09 (s, 3H), 2.95 (s, 3H), 2.93 - 2.84 (m, 1H), 2.04 (dtd, J = 23.4, 13.2, 11.4, 6.1 Hz, 2H), 1.83 (dt, J = 10.0, 5.9 Hz, 2H), 1.69 (d, J = 10.1 Hz, 2H).

¹³C NMR (101 MHz, CDCl₃) δ 173.92, 158.32, 157.58, 148.12, 147.17, 138.07, 128.02, 114.62, 60.42, 50.05, 37.17, 35.68, 33.47, 31.63, 27.27, 26.17.

LCMS: m/z=393.8 (M+H)⁺.

### Example 3

### Preparation of N1-((1S,2R,4S)-2-azido-4-(dimethylcarbamoyl)cyclohexyl)-N2-(5-chloropyridin-2-yl) oxalamide (a compound of a formula 1)

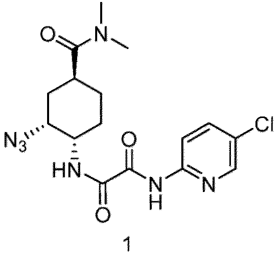

Ethyl 2-((5-chloropyridin-2-yl)amino)-2-oxoacetate (a compound of a formula 3) (150.7 g, 568.7 mmol), and 500 mL of acetonitrile were sequentially added into a reaction flask; and a reaction system was cooled to about 0-10°C, triethylamine (43.1 g, 426.7 mmol) was added slowly, (1S,3R,4S)-4-amino-3-azido-N,N-dimethylcyclohexane-1-carboxamide (a compound of a formula 2) (100.0 g, 473.9 mmol) was then added, after the addition was completed, the reaction system was heated to 60-65°C, triethylamine (14.4 g, 142.6 mmol) was then added into the system, and stirring was then performed for 16 h while maintaining the temperature of the system at 60-65°C. After completion of the reaction by liquid phase monitoring, 900 mL of purified water was added dropwise to the system, after the addition was completed, cooling was performed to 20-25°C, stirring was performed for 1 h, and suction filtration was performed to obtain 171.4 g of a compound (the compound of the formula 1), with a yield of 92.0%.

H NMR data of the obtained product was as follows:
¹H NMR (400 MHz, CDCl₃) δ 9.79 (s, 1H), 8.32 (d, J = 2.4 Hz, 1H), 8.19 (d, J = 8.8 Hz, 1H), 7.72 (dd, J = 8.9, 2.4 Hz, 1H), 7.63 (d, J = 8.8 Hz, 1H), 4.13 (d, J = 3.4 Hz, 1H), 4.02 (td, J = 9.7, 8.9, 4.7 Hz, 1H), 3.09 (s, 3H), 2.95 (s, 3H), 2.93 - 2.84 (m, 1H), 2.04 (dtd, J = 23.4, 13.2, 11.4, 6.1 Hz, 2H), 1.83 (dt, J = 10.0, 5.9 Hz, 2H), 1.69 (d, J = 10.1 Hz, 2H).

¹³C NMR (101 MHz, CDCl₃) δ 173.92, 158.32, 157.58, 148.12, 147.17, 138.07, 128.02, 114.62, 60.42, 50.05, 37.17, 35.68, 33.47, 31.63, 27.27, 26.17.

LCMS: m/z=393.8 (M+H)⁺.

### Example 4

### N1-((1S,2R,4S)-2-amino-4-(dimethylcarbamoyl)cyclohexyl)-N2-(5-chloropyridi n-2-yl)oxalamide (a compound of a formula 4)

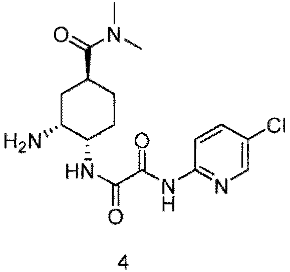

N1-((1S,2R,4S)-2-azido-4-(dimethylcarbamoyl)cyclohexyl)-N2-(5-chloropyridin -2-yl)oxalamide (the compound of the formula 1) (5.0 g, 12.7 mmol), Pd/C (0.1 g, 2%wt) and 40 mL of methanol were sequentially added into a hydrogen reactor, and a reaction was carried out while maintaining at 50-55°C for 8 hours. After completion of the reaction by TLC monitoring, an insoluble substance was filtered off, a filter cake was washed with 2×20 mL of methanol, and an organic phase was concentrated to give a compound (the compound of the formula 4) which was directly used in a next step according to a yield of 100%.

H NMR data of the obtained product was as follows:
¹H NMR (400 MHz, CDCl₃) δ 8.30 (d, J = 2.3 Hz, 1H), 8.17 (dd, J = 8.9, 2.1 Hz, 1H), 7.98 (d, J = 8.5 Hz, 1H), 7.70 (dt, J = 8.9, 2.3 Hz, 1H), 3.91 (dt, J = 13.0, 6.5 Hz, 1H), 3.35 (d, J = 4.7 Hz, 1H), 3.06 (d, J = 2.1 Hz, 3H), 2.93 (d, J = 2.1 Hz, 3H), 2.89 - 2.79 (m, 1H), 1.96 (tt, J = 12.3, 2.8 Hz, 1H), 1.80 (q, J = 12.6, 11.2 Hz, 3H), 1.73 - 1.54 (m, 3H).

¹³C NMR (101 MHz, CDCl₃) δ 174.59, 158.24, 158.09, 148.27, 147.14, 138.02, 127.86, 114.52, 50.88, 47.88, 37.10, 35.67(overlapped), 32.84, 27.24, 25.24.

LCMS: m/z=367.8 (M+H)⁺.

### Example 5

### Preparation of N1-((1S,2R,4S)-2-amino-4-(dimethylcarbamoyl)cyclohexyl)-N2-(5-chloropyridin-2-y l)oxalamide (a compound of a formula 4)

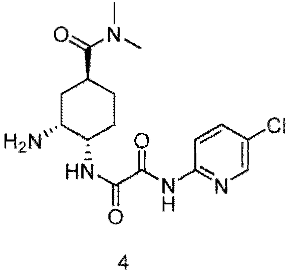

N1-((1S,2R,4S)-2-azido-4-(dimethylcarbamoyl)cyclohexyl)-N2-(5-chloropyridin -2-yl)oxalamide (1) (5.0 g, 12.7 mmol), triphenylphosphine (6.6 g, 25.4 mmol), 30 mL of tetrahydrofuran and 10 mL of purified water were sequentially added into a reaction flask, stirring was performed overnight at 20-25°C, after completion of the reaction by TLC monitoring, IN HCl was added into the system to adjust a pH to be 2-3, stirring was performed for 1 h while maintaining at room temperature, then concentration was performed to remove tetrahydrofuran, an aqueous phase was washed with 2×30 mL of dichloromethane, aqueous phases were retained, 2N sodium hydroxide was added to adjust a pH to be 9, the aqueous phase was extracted with 2×30 mL of dichloromethane, the mixed organic phase was concentrated to obtain a compound (the compound of the formula 4) which was directly used in a next step according to a yield of 100%.

H NMR data of the obtained product was as follows:
¹H NMR (400 MHz, CDCl₃) δ 8.30 (d, J = 2.3 Hz, 1H), 8.17 (dd, J = 8.9, 2.1 Hz, 1H), 7.98 (d, J = 8.5 Hz, 1H), 7.70 (dt, J = 8.9, 2.3 Hz, 1H), 3.91 (dt, J = 13.0, 6.5 Hz, 1H), 3.35 (d, J = 4.7 Hz, 1H), 3.06 (d, J = 2.1 Hz, 3H), 2.93 (d, J = 2.1 Hz, 3H), 2.89 - 2.79 (m, 1H), 1.96 (tt, J = 12.3, 2.8 Hz, 1H), 1.80 (q, J = 12.6, 11.2 Hz, 3H), 1.73 - 1.54 (m, 3H).

¹³C NMR (101 MHz, CDCl₃) δ 174.59, 158.24, 158.09, 148.27, 147.14, 138.02, 127.86, 114.52, 50.88, 47.88, 37.10, 35.67(overlapped), 32.84, 27.24, 25.24.

LCMS: m/z=367.8 (M+H)⁺.

### Example 6

### Preparation of Edoxaban (a compound of a formula 6)

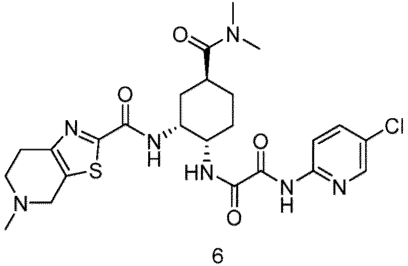

N1-((1S,2R,4S)-2-amino-4-(dimethylcarbamoyl)cyclohexyl)-N2-(5-chloropyridi n-2-yl)oxalamide (the compound of the formula 4) (5.0 g, 13.6 mmol) and 75 mL acetonitrile were added into a reaction flask, a reaction system was cooled to about 0-10°C under stirring, triethylamine (1.7 g, 16.3 mmol), 4,5,6,7-tetrahydro-5-methyl-thiazolo[5,4-c]pyridine-2-carboxylic acid (5) (3.5 g, 15.0 mmol), HOBT (2.2 g, 16.3 mmol), and EDCI (3.6 g, 19.0 mmol) were then sequentially added, heating was performed to 20-25°C, stirring was performed for 8 h, after completion of the reaction by liquid phase monitoring, suction filtration was performed, a filter cake was taken out to be dissolved in 50 mL of dichloromethane, an organic phase was washed with 2×25 mL of a saturated aqueous sodium carbonate solution, the organic phase was concentrated, 30 mL of absolute ethanol was added, hot slurrying was performed at 70-75°C for 2 h, cooling was performed to room temperature, and suction filtration was performed to give 6.3 g of a compound (the compound of the formula 6), with a yield of 85%.

H NMR data of the obtained product was as follows:
¹H NMR (400 MHz, CDCl₃) δ 9.76 (s, 1H), 8.32 (dd, J = 2.4, 0.6 Hz, 1H), 8.18 (dd, J = 8.8, 0.6 Hz, 1H), 8.04 (d, J = 7.8 Hz, 1H), 7.71 (dd, J = 8.8, 2.4 Hz, 1H), 7.42 (dd, J = 8.8, 0.6 Hz, 1H), 4.66 - 4.71 (m, 1H), 4.07 - 4.13 (m, 1H), 3.78 (d, J = 15.1 Hz, 2H), 3.08 (s, 3H), 3.02 - 2.79 (m, 8H), 2.58 (q, J = 6.2 Hz, 3H), 2.11 (m, 3H), 2.01 - 1.60 (m, 3H).

¹³C NMR (101 MHz, CDCl₃) δ 173.59, 160.26, 159.92, 158.77, 157.68, 156.55, 149.90, 148.17, 147.12, 137.94, 127.88, 114.58, 52.49, 52.15, 51.01, 47.79, 45.00, 37.18, 35.76, 34.09, 32.64, 26.99, 26.66, 25.97.

LCMS: m/z=547.8 (M+H)⁺.

### Example 7

### Preparation of Edoxaban (a compound of a formula 6)

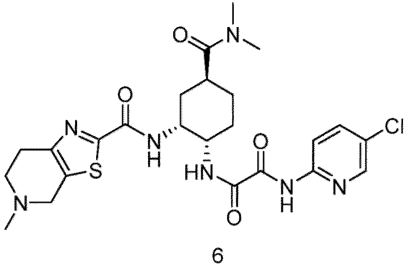

4,5,6,7-Tetrahydro-5-methyl-thiazolo[5,4-C]pyridine-2-carboxylic acid (a compound of a formula 5) (3.8 g, 16.3 mmol) and 30 mL of dichloromethane were sequentially added into a reaction flask, and oxalyl chloride (2.6 g, 20.4 mmol) was slowly added dropwise into the system while maintaining at 20-25°C under nitrogen protection. After the addition was completed, the mixture was stirred for 2 h under nitrogen while maintaining at room temperature, suction filtration was performed, and washing was performed with dichloromethane to obtain 4,5,6,7-tetrahydro-5-methyl-thiazolo[5,4-C]pyridine-2-carbonyl chloride hydrochloride which was stored for standby application.

N1-((1S,2R,4S)-2-amino-4-(dimethylcarbamoyl)cyclohexyl)-N2-(5-chloropyridi n-2-yl)oxalamide (the compound of the formula 4) (5.0 g, 13.6 mmol), 75 mL of dichloromethane and triethylamine (3.4 g, 34.0 mmol) were sequentially added into another reaction flask; and a reaction system was cooled to 0-5°C under stirring, the prepared 4,5,6,7-tetrahydro-5-methyl-thiazolo[5,4-C]pyridine-2-carbonyl chloride hydrochloride was added slowly, after the addition was completed, heating was performed to room temperature, stirring was performed for 2-3 h, after completion of the reaction by liquid phase monitoring, an organic phase was washed with 2×25 mL of a saturated aqueous sodium carbonate solution, 30 mL of absolute ethanol was added, after dichloromethane was distilled off at atmospheric pressure, a solid was precipitated, and suction filtration was performed to give 6.6 g of a compound (the compound of the formula 6), with a yield of 88.6%.

H NMR data of the obtained product was as follows:
¹H NMR (400 MHz, CDCl₃) δ 9.76 (s, 1H), 8.32 (dd, J = 2.4, 0.6 Hz, 1H), 8.18 (dd, J = 8.8, 0.6 Hz, 1H), 8.04 (d, J = 7.8 Hz, 1H), 7.71 (dd, J = 8.8, 2.4 Hz, 1H), 7.42 (dd, J = 8.8, 0.6 Hz, 1H), 4.66 - 4.71 (m, 1H), 4.07 - 4.13 (m, 1H), 3.78 (d, J = 15.1 Hz, 2H), 3.08 (s, 3H), 3.02 - 2.79 (m, 8H), 2.58 (q, J = 6.2 Hz, 3H), 2.11 (m, 3H), 2.01 - 1.60 (m, 3H).

¹³C NMR (101 MHz, CDCl₃) δ 173.59, 160.26, 159.92, 158.77, 157.68, 156.55, 149.90, 148.17, 147.12, 137.94, 127.88, 114.58, 52.49, 52.15, 51.01, 47.79, 45.00, 37.18, 35.76, 34.09, 32.64, 26.99, 26.66, 25.97.

LCMS: m/z=547.8 (M+H)⁺.

### Example 8

### Preparation of edoxaban p-toluenesulfonate monohydrate (a compound of a formula 7)

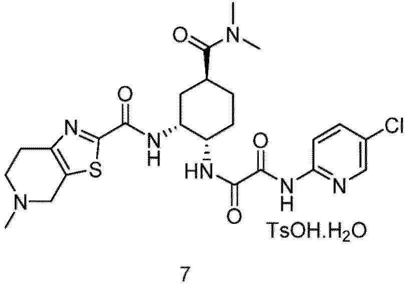

Edoxaban (the compound of the formula 6) (5.0 g, 9.1 mmol) and 50 mL of dichloromethane were sequentially added into a reaction flask, a 1 mol/L solution of p-toluenesulfonic acid in acetone (9.1 mL) was added into the above solution, and a solvent was distilled off. Acetone containing 15% water (50 mL) was added into a residue, and the obtained mixture was stirred at 60°C to dissolve the mixture. The obtained solution was cooled to room temperature and stirred for 1 day. Precipitated crystals were filtered and collected, washed with acetone and dried under reduced pressure at room temperature for 2 h to give 6.0 g of a compound (the compound of the formula 7), with a yield of 89%.

H NMR data of the obtained product was as follows:
¹H NMR (400 MHz, DMSO-d₆) δ 10.29 (s, 1H), 10.20 (s, 1H), 9.20 (d, J = 8.1 Hz, 1H), 8.76 (d, J = 7.4 Hz, 1H), 8.48 - 8.41 (m, 1H), 8.05 - 7.97 (m, 2H), 7.45 (dd, J = 8.0, 1.6 Hz, 2H), 7.10 (d, J = 7.7 Hz, 2H), 4.61 (s, 2H), 4.43 (dt, J = 7.5, 3.9 Hz, 1H), 4.01 (ddt, J = 12.6, 8.5, 4.2 Hz, 1H), 3.64 (s, 2H), 3.18 (q, J = 5.5 Hz, 2H), 2.99 (s, 3H), 2.93 (s, 4H), 2.78 (s, 3H), 2.27 (s, 3H), 2.15 - 1.98 (m, 2H), 1.82 - 1.62 (m, 3H), 1.56 - 1.42 (m, 1H).

¹³C NMR (101 MHz, DMSO-d₆) δ 174.22, 163.73, 160.05, 159.20, 158.83, 149.06, 148.33, 147.33, 145.49, 138.77, 138.44, 128.61, 127.33, 127.16, 125.86, 115.49, 113.74, 100.48, 50.94, 50.85, 49.98, 48.60, 42.29, 36.95, 35.41, 33.38, 31.81, 27.18, 25.37, 24.16, 21.20.

LCMS: m/z=547.8 (M+H)⁺.

### Example 9

### Preparation of edoxaban p-toluenesulfonate monohydrate (a compound of a formula 7)

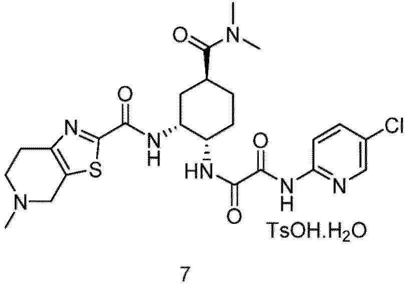

Edoxaban (the compound of the formula 6) (5.0 g, 9.1 mmol), 25 mL of absolute ethanol and 10 mL of purified water were sequentially added into a reaction flask, p-toluenesulfonic acid monohydrate (1.6 g, 8.4 mmol) was added into the system, the temperature was raised to 70-75°C, the mixture was stirred until the obtained solution was clear, then the temperature was lowered, and when the system was cooled to 55-60°C, p-toluenesulfonic acid monohydrate (0.3 g, 1.6 mmol) was supplementarily added to the system, after the addition was completed, 50 mL of absolute ethanol was added dropwise into the system, then the temperature was lowered to 20-25°C, stirring was performed for 1 h, then the temperature was lowered to 0-10°C, stirring was performed for 1 h, suction filtration was performed, washing was performed with 2×10 mL of absolute ethanol, and drying was performed under reduced pressure at 45°C to give 6.2 g of a compound (the compound of the formula 7), with a yield of 92%.

H NMR data of the obtained product was as follows:
¹H NMR (400 MHz, DMSO-d₆) δ 10.29 (s, 1H), 10.20 (s, 1H), 9.20 (d, J = 8.1 Hz, 1H), 8.76 (d, J = 7.4 Hz, 1H), 8.48 - 8.41 (m, 1H), 8.05 - 7.97 (m, 2H), 7.45 (dd, J = 8.0, 1.6 Hz, 2H), 7.10 (d, J = 7.7 Hz, 2H), 4.61 (s, 2H), 4.43 (dt, J = 7.5, 3.9 Hz, 1H), 4.01 (ddt, J = 12.6, 8.5, 4.2 Hz, 1H), 3.64 (s, 2H), 3.18 (q, J = 5.5 Hz, 2H), 2.99 (s, 3H), 2.93 (s, 4H), 2.78 (s, 3H), 2.27 (s, 3H), 2.15 - 1.98 (m, 2H), 1.82 - 1.62 (m, 3H), 1.56 - 1.42 (m, 1H).

¹³C NMR (101 MHz, DMSO-d₆) δ 174.22, 163.73, 160.05, 159.20, 158.83, 149.06, 148.33, 147.33, 145.49, 138.77, 138.44, 128.61, 127.33, 127.16, 125.86, 115.49, 113.74, 100.48, 50.94, 50.85, 49.98, 48.60, 42.29, 36.95, 35.41, 33.38, 31.81, 27.18, 25.37, 24.16, 21.20.

LCMS: m/z=547.8 (M+H)⁺.

## Claims

1. An edoxaban key intermediate, **characterized by** having a structural formula shown in a formula 1:

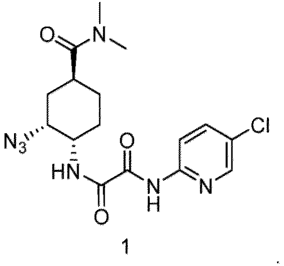

2. A synthesis method for the edoxaban key intermediate according to claim 1, **characterized in that** the edoxaban key intermediate is obtained by an ammonolysis reaction between a compound of a formula 2 and a compound of a formula 3, and a synthetic route is as follows:

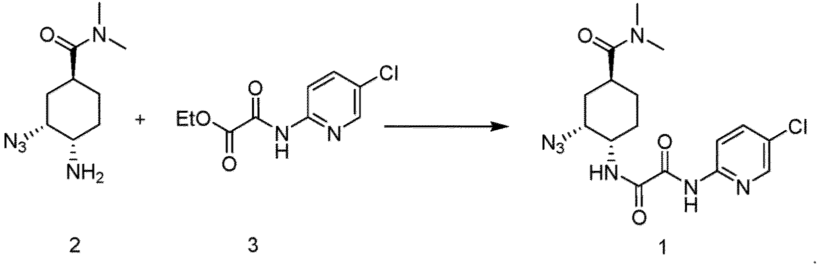

3. The synthesis method for the edoxaban key intermediate according to claim 2, **characterized in that** a solvent used in the ammonolysis reaction is at least one of acetonitrile, tetrahydrofuran, ethanol, toluene, and butanone; and an alkali used in the ammonolysis reaction is at least one of triethylamine, diisopropylethylamine, pyridine, and DBU.

4. The synthesis method for the edoxaban key intermediate according to claim 2, **characterized in that** the ammonolysis reaction is carried out at a temperature of 50-100°C.

5. The synthesis method for the edoxaban key intermediate according to claim 2, **characterized in that** a molar ratio of the compound of the formula 2 to the compound of the formula 3 is 1:1.1-1.5.

6. Use of the edoxaban key intermediate according to claim 1, **characterized by** being used for the preparation of edoxaban p-toluenesulfonate monohydrate, specifically comprising the steps of:
step 1): subjecting a compound of a formula 1 to a reduction reaction to obtain a compound of a formula 4, wherein a synthetic route is as follows: 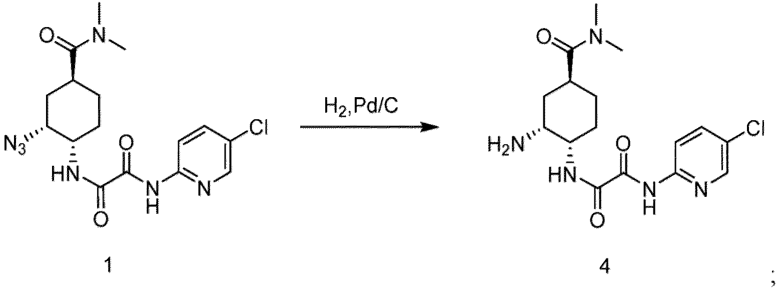
step 2): subjecting the compound of the formula 4 to a condensation reaction to obtain a compound of a formula 6, wherein a synthetic route is as follows: 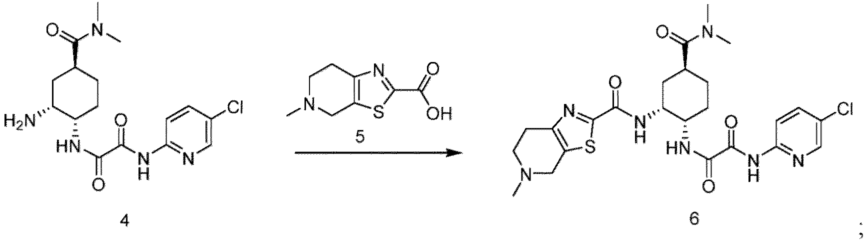 and
step 3): performing salt formation by using the compound of the formula 6 and p-toluenesulfonic acid to obtain edoxaban p-toluenesulfonate monohydrate, i.e., a compound of a formula 7:

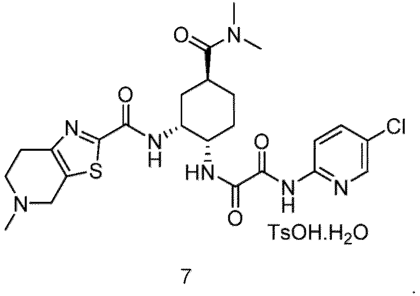

7. The use according to claim 6, **characterized in that** a reducing agent used in the step 1) is at least one of Pd/C, Raney nickel, lithium aluminum hydride, sodium borohydride, and triphenylphosphine.

8. The use according to claim 6, **characterized in that** a solvent used in the condensation reaction of the step 2) is at least one of acetonitrile, ethanol, tetrahydrofuran, and dichloromethane.

9. The use according to claim 6, **characterized in that** in the step 2), a compound of a formula 5 is prepared into acyl chloride for the reaction or a condensation reagent is used for the reaction, the condensation reagent being at least one of CDI, DCC, and EDCI.

10. The use according to claim 6, **characterized in that** the step 3) is carried out in a reaction solvent, and the reaction solvent is at least one of ethanol, acetonitrile, and acetone; the temperature is controlled to be 50-100°C in the step 3); and the temperature of the salt formation in the step 3) is -10°C to 30°C.
